# FASCICULE DE BREVET EUROPEEN

(11) **EP 0 953 317 B1**
(45) Date de publication et mention de la délivrance du brevet: **07.04.2004**
(21) Numéro de dépôt: 99401041.1
(22) Date de dépôt: 29.04.1999
(51) Int. Cl.: A61B 17/70

(54) **Implant squelettique**
Skelettimplantat
Skeletal implant

(30) Priorité: 30.04.1998 FR 9805549
(43) Date de publication de la demande: 03.11.1999
(73) Titulaire: Zacouto, Fred, 75015 Paris (FR)
(72) Inventeur: Zacouto, Fred, 75015 Paris (FR); Caballero, Efren Vigil, 33212 Gijon (ES); Canal, Jose Angel Alvarez, 33930 La Feiguera (ES)
(74) Mandataire: Jacobson, Claude

(56) Documents cités:
- EP-A- 0 611 554
- EP-A- 0 820 731
- EP-A- 0 821 917
- WO-A-94/06364
- FR-A- 2 755 844
- GB-A- 2 283 178
- US-A- 4 932 975
- US-A- 5 720 746

## Description

La présente invention concerne un implant squelettique et notamment un implant destiné à assurer des liaisons
entre au moins deux éléments du squelette humain, notamment deux vertèbres, ledit implant pouvant être simple ou multiple et composé, par exemple, de deux ou plusieurs implants individuels.

Un nouvel implant squelettique, utilisable pour assurer une liaison entre au moins deux éléments du squelette humain, et notamment deux vertèbres, a été décrit dans la demande EP-A 0820731 et la demande US Serial number 08/897 673

Ces demandes décrivent des implants qui se caractérisent, notamment, par le fait qu'ils possèdent une longueur ou dimension variable, les deux extrémités de l'implant étant susceptibles de s'éloigner ou de se rapprocher l'une de l'autre activement et/ou passivement, par exemple, suivant l'axe longitudinal de l'implant, grâce à l'interposition d'un élément déformable, par exemple un élément hydraulique, des moyens de commande et/ou de régulation étant prévus, de façon à permettre d'obtenir un changement de dimension de l'implant pour modifier la distance entre les deux éléments osseux et/ou pour assurer un amortissement visqueux ou viscoélastique ajustable autorisant un mouvement lent entre les deux éléments osseux et s'opposant davantage à un déplacement plus brusque.

Si nécessaire, le maintien d'une haute pression hydraulique suffisante peut être assuré en utilisant un effet de pompe mécanique, actionné par les mouvements du corps, avec clapet de limitation de pression, coopérant avec un réservoir basse pression.

Dans cette demande sont décrits, par exemple, des implants doubles, constitués de deux implants individuels susceptibles d'être disposés respectivement des deux côtés du rachis pour relier deux vertèbres, chacun des deux éléments étant ainsi fixé à une vertèbre inférieure par un moyen d'ancrage, tel qu'une vis pédiculaire, et à une vertèbre supérieure, adjacente ou plus éloignée, par également chaque fois un moyen d'ancrage, tel qu'une vis pédiculaire.

Dans le cas d'un implant double composé de deux implants individuels agissant sur les mêmes structures squelettiques, il a également déjà été prévu dans lesdites demandes EP-A-082031 et US 08/897 673 d'interconnecter de façon hydraulique les deux implants individuels de façon à autoriser des mouvements de pivotement des éléments osseux l'un par rapport à l'autre, à savoir un pivotement latéral dans le plan frontal du rachis, par augmentation de longueur de l'un des implants individuels et diminution concomitante de longueur de l'autre implant, pour une correction de déformation et/ou un amortissement de flexion latérale.

Grâce à des moyens de commande, par exemple des moyens non invasifs de type magnétique, on peut assurer les modifications de dimension désirée des implants individuels et/ou les modifications du coefficient d'amortissement de l'élément jouant un rôle d'amortisseur. De plus, il peut être prévu des moyens modifiant automatiquement le coefficient d'amortissement ou la viscosité en fonction des mouvements corporels.

Le document EP-A-0 821917 décrit un implant intervertébral comprenant deux vis pédiculaires montées sur des rotules. Entre les deux vis, sont prévus des coussins solides amortisseurs destinés à amortir des mouvements de vibration et autoriser une rotation passive, cet implant étant d'ailleurs destiné à réaliser une solidarisation destinée à bloquer entre elles les parties osseuses auxquelles il est fixé. Il en résulte que seul un amortissement passif de vibrations est autorisé.

Comme le document précédent, le document EP-A-0 611554 décrit une prothèse intervertébrale extra discale présentant deux vis pédiculaires avec interposition de coussins amortisseurs destinés à amortir les vibrations en autorisant un déplacement passif pour la régulation du mouvement de flexion/extension entre deux vertèbres reliées par l'implant en développant une course exponentielle dans les deux directions de manière à se rapprocher le plus possible de l'allure physiologique d'un mouvement discal.

Dans le document EP-A-0 820731, des mouvements de basculements sont également possibles en entraînant notamment un amortissement.

Ces documents ne décrivent cependant pas un implant unique et permettant d'imposer, en fonction des besoins physiologiques, des mouvements et des efforts de rotation complexes entre des parties osseuses auxquelles est fixé l'implant.

Le document WO94/06364 décrit un fixateur externe de consolidation osseuse dans lequel peut exister un mouvement de translation.

La présente invention permet de réaliser et de perfectionner un dispositif implantable comprenant au moins un implant muni de deux extrémités susceptibles d'être fixées, par des moyens d'ancrage, sur au moins deux portions ou parties de squelette et comprenant des moyens de déplacement, de préférence au moins partiellement réversible, entre lesdites deux extrémités, agencés pour provoquer/ou maintenir un déplacement entre lesdites portions ou éléments de squelette.

Ce déplacement peut un déplacement rectiligne et/ou courbe, par exemple être un déplacement d'allongement, encore appelé de distraction, ou un déplacement de raccourcissement, dit de compression, ou un déplacement en rotation, cette rotation pouvant être isolée ou au contraire associée à une distraction ou une compression.

L'invention a pour objet un implant squelettique, présentant un premier et un second élément d'extrémité (2, 3, 15, 16, 21, 22, 27, 28, 34, 38, 51, 52) des moyens d'ancrage (10, 11) susceptibles d'être fixés dans des parties osseuses, reliés respectivement auxdits premier et second éléments d'extrémité, au moins un élément déformable (4, 17, 23, 32, 39, 57) relié respectivement auxdits premier et second éléments d'extrémité et autorisant un mouvement entre eux, et donc entre lesdits moyens d'ancrage, caractérisé en ce qu'il comporte des moyens d'articulation (5, 6, 8, 9, 24, 25, 31, 37, 60) autorisant une rotation desdits moyens d'ancrage autour d'un axe perpendiculaire et/ou d'un axe parallèle à la direction géométrique rejoignant lesdits éléments d'extrémité, et en ce qu'il comporte un moyen moteur dudit élément déformable pour provoquer sa déformation, entraînant ladite rotation.

Ledit moyen moteur est, de préférence, un moyen hydraulique.

Lesdits moyens de déplacement sont capables de maintenir, mais non de provoquer le déplacement, ces moyens de déplacements sont contrôlés par des moyens de commande, de préférence non invasifs, qui permettent de les libérer de façon à permettre au patient, ou à un intervenant, de modifier la position relative des deux portions ou éléments de squelette, après quoi lesdits moyens de commande sont actionnés pour bloquer l'implant dans cette nouvelle position, un déplacement en sens inverse ou réversible restant possible si l'on agit à nouveau sur les moyens de commande, par exemple dans le cas où les déplacements auraient été trop importants.

Ledit moyen moteur permet d'imposer un déplacement entre lesdites extrémités en exerçant une force entre elles.

Dans une forme de réalisation particulière, cette force peut être exercée temporairement, c'est-à-dire pendant un instant assez court, dans le but de provoquer un déplacement thérapeutiquement désirable entre les deux portions ou éléments de squelette, un tel déplacement étant souvent prévu pour une faible amplitude, car rapidement contrecarré par les structures anatomiques qu'il convient de pas traumatiser. A la fin de cet instant, les moyens de commande permettent de bloquer les deux extrémités l'une par rapport à l'autre et de maintenir l'implant dans sa nouvelle position.

Dans une autre forme de réalisation, au contraire, lesdits moyens de déplacement sont capables d'exercer une force permanente anatomiquement active entre lesdites deux extrémités, cette force pouvant être constante ou variable, de façon à exercer, sur l'environnement anatomique, une contrainte qui va progressivement autoriser un déplacement anatomiquement souhaité entre lesdites deux portions ou éléments de squelette, ces moyens moteurs étant commandables par des moyens de commande permettant d'autoriser ou d'interrompre leur fonctionnement, et/ou d'ajuster l'intensité de la force.

Eventuellement, l'implant peut également comprendre des moyens d'amortissement visqueux ou viscoélastiques pouvant être mis en oeuvre lorsque l'implant est bloqué dans sa dimension ou lorsque l'implant est libéré ou lorsqu'il exerce sa force active permanente. De tels moyens ont été décrits dans les demandes européenne et américaine précitées.

Lesdits moyens de déplacement et, s'il y a lieu, lesdits moyens moteurs peuvent être de type hydraulique, et/ou mécanique, et/ou électrique, une réalisation hydraulique étant préférée.

Dans une forme de réalisation utilisant des moyens hydrauliques, l'implant comporte préférentiellement :
deux éléments d'extrémité, par exemple des tiges, recevant chacun au moins un moyen d'ancrage dans une partie squelettique,
au moins un élément déformable, de préférence hydraulique, interposé entre lesdits deux éléments, et permettant une variation dimensionnelle et/ou l'établissement d'une force active entre eux ;
de préférence, au moins un réservoir haute pression, dit de réserve, permettant d'adresser la haute pression, à la demande à un circuit utilisateur fonctionnel ;
de préférence au moins un réservoir collecteur basse pression relié au réservoir haute pression par une valve régulatrice de pression ;
de préférence, au moins un circuit de recharge du réservoir haute pression comprenant au moins un élément déformable, de préférence sensible à des mouvements ou positions corporelles de l'organisme recevant l'implant, pour engendrer une pression élevée permettant, en cas de besoin, l'alimentation du réservoir haute pression ;
au moins un circuit fonctionnel, à savoir :
un circuit de modification de dimension, par exemple de longueur, d'implant comprenant ledit élément déformable permettant de modifier une dimension entre les deux éléments d'extrémité et/ou d'établir, entre les deux éléments d'extrémité, une force active susceptible de provoquer une modification, progressive, de dimension entre lesdits éléments d'extrémité, ledit élément déformable étant relié, d'une part, au réservoir de réserve haute pression par l'intermédiaire d'une première vanne et, d'autre part, au réservoir collecteur basse pression, par l'intermédiaire d'une deuxième vanne pour permettre, en fonction de la commande desdites vannes, d'augmenter et/ou de diminuer la dimension dudit élément déformable pour permettre ou provoquer une modification durable de ladite dimension dudit implant individuel,
   et/ou
un circuit d'amortissement visqueux ou viscoélastique comprenant :
   éventuellement un élément élastique opérationnellement interposé entre lesdits deux éléments d'extrémités d'implant, et un élément amortisseur hydraulique comprenant au moins un élément déformable sensible à la vitesse d'une variation dimensionnelle de l'implant et communiquant avec un réservoir de décharge par l'intermédiaire d'un moyen d'étranglement,
      - et des moyens de commande, de préférence, actionnables depuis l'extérieur du corps du patient, pour modifier la dimension de l'implant, et/ou la force exercée par l'implant et/ou les propriétés d'amortissement.

Bien entendu une même pièce, par exemple un élément déformable, peut faire partie constitutive de plusieurs des constituants définis ci-dessus.

Le circuit de recharge du réservoir haute pression est destinée à jouer, en fait un rôle de pompe très haute pression permettant d'établir et de maintenir une pression élevée dans un réservoir haute pression.

De préférence, notamment dans le cas, préféré, où l'élément déformable du circuit de recharge haute pression est sensible à des mouvements ou des positions corporels du patient recevant l'implant, on prévoit que cet élément déformable ait une petite surface, comparé à la surface active de l'élément qui lui transmet la force en provenance de l'organisme, de façon à assurer un effet différentiel multiplicateur de pression, étant entendu que l'on peut admettre qu'à chaque sollicitation, seule une faible quantité de fluide sous très haute pression est adressée vers la chambre haute pression.

L'élément déformable du circuit de recharge du réservoir haute pression peut aussi être actionné par des moyens extérieurs tout en restant implanté. Ainsi, par exemple, cet élément déformable peut se présenter sous forme d'une pompe, de préférence réalisée par un soufflet métallique, implanté sur une partie corporelle à un niveau où on peut lui appliquer une pression externe, par exemple implanté sur la face postérieure du sacrum, permettant à la main d'un intervenant, à travers les plans anatomiques externes, d'actionner cette pompe ou soufflet.

En variante, cette pompe pourrait être de type magnétique ou électromagnétique, en possédant, par exemple, un noyau mobile actionnant un petit piston ou soufflet sous l'influence d'une force électromagnétique d'origine externe.

On comprend également que l'on incorpore, dans la présente demande, les variantes et équivalents utilisant des moyens moteurs et/ou amortisseurs, non-hydrauliques assurant les mêmes fonctions de modification durable et ajustable de dimension et/ou de force et/ou de modification ajustable ou variation de coefficient d'amortissement.

Uniquement à titre d'exemple, un implant de type uniquement mécanique peut comprendre une première extrémité, mobile en translation par rapport à la deuxième extrémité, et solidaire d'une tige immobilisée par un cliquet sensible à un moyen de commande magnétique externe pour bloquer ou libérer ladite tige, un moyen moteur étant interposé entre ladite tige et ladite deuxième extrémité, ce moyen moteur pouvant être réalisé sous forme d'un ressort ou d'un autre élément élastique préchargé tendant à déplacer l'une des extrémités par rapport à l'autre lorsque l'on libère le cliquet, le mouvement pouvant être réversible, au moins une fois, par exemple, en intercalant entre le ressort et ladite deuxième extrémité une pièce d'appui de ressort que l'on peut déplacer, grâce à d'autres moyens de commande magnétique, de façon à détendre au moins partiellement le ressort. En variante, l'implant peut comporter plusieurs ressorts disposés parallèlement et susceptibles d'être mis en oeuvre séparément par des moyens de libération sensibles à des moyens de commande.

Dans une autre forme de réalisation, un implant peut comporter des moyens de déplacement de type électromagnétique, par exemple un solénoïde avec un noyau plongeur, le solénoïde étant solidaire d'une des extrémités d'un implant et le noyau plongeur de l'autre extrémité, des moyens de blocage étant, de préférence prévus pour immobiliser le noyau par rapport au solénoïde dans au moins deux postions différentes, le solénoïde étant susceptible d'être alimenté, par un moyen de commande, à partir d'une source d'énergie électrique, par exemple une pile implantée et/ou un accumulateur rechargeable par transmission d'antenne à couplage transcutané.

De telles réalisations sont réversibles au sens de l'invention car elles permettent, si on le souhaite, d'obtenir une modification en sens inverse, au moins en partie, de la modification dimensionnelle établie.

La présente invention permet également de perfectionner les mouvements ou forces de rotation autorisés ou imposés par un implant ou un ensemble d'au moins deux implants individuels, dans le plan frontal et/ou sagittal et/ou horizontal.

Par exemple l'invention permet de fournir des implants de ce type permettant d'imposer des mouvements de rotation symétriques, c'est-à-dire de même sens et de même valeur de rotation des vis pédiculaires ou moyens d'ancrage similaires des deux éléments d'implant individuel, ou au contraire des mouvements antisymétriques, c'est-à-dire de sens opposés, ou encore indépendants l'un de l'autre.
D'une façon générale, le mouvement ou force de rotation contrôlé entre les deux moyens d'ancrage d'un implant selon l'invention, et le cas échéant la coordination des mouvements ou forces de rotation des moyens d'ancrage de plusieurs implants, par exemple les implants individuels d'un implant double, permettra, selon les besoins, de se rapprocher beaucoup plus du mouvement naturel théoriquement possible ou souhaitable entre les deux parties osseuses auxquelles sont fixées les moyens d'ancrage, pour imposer des déplacements progressifs, par exemple pour des corrections, et/ou des amortissements progressivement modifiables, partant par exemple de la rigidité pendant une phase de consolidation osseuse ou de cicatrisation vers une mobilité progressive, permettant, par exemple, de sauvegarder une articulation.

L'implant squelettique présente un premier et un second élément d'extrémité, des moyens d'ancrage dans des parties osseuses reliés respectivement auxdits premier et second éléments d'extrémité, au moins un élément déformable relié respectivement auxdits premier et second moyens d'ancrage, et des moyens autorisant un mouvement non rectiligne, notamment une rotation entre lesdits moyens d'ancrage

L'implant peut comporter des moyens autorisant une rotation entre lesdits éléments d'extrémité.

Ledit élément déformable peut être déformable en rotation.

L'implant peut comporter des moyens autorisant une rotation d'au moins un desdits moyens d'ancrage par rapport à la pièce d'extrémité à laquelle il est relié.

Le mouvement de rotation précité peut aussi être associé à des mouvements en translation, de sorte que le moùvement résultant peut être un déplacement complexe non rectiligne.

Selon un perfectionnement, l'implant squelettique, présentant un premier et un deuxième élément d'extrémité, des moyens d'ancrage dans des parties osseuses, par exemple par l'intermédiaire de vis, telles que par exemple des vis pédiculaires situées auxdites extrémités, au moins un élément déformable, par exemple, un élément hydraulique, contenant un fluide hydraulique indéformable, et interposé entre les deux éléments d'extrémité, et des moyens d'actionnement de l'élément déformable, par exemple, s'il y a lieu, des moyens de circuit hydraulique reliés audit au moins un élément déformable interposé entre lesdites extrémités, et susceptibles de permettre une modification durable, de préférence obtenue progressivement, de la distance ou de la force entre lesdites deux extrémités et/ou un amortissement viscoélastique des mouvements entre lesdites deux extrémités, lesdits moyens d'actionnement, par exemple lesdits moyens de circuit hydraulique, étant sensibles à des moyens de commande, de préférence, actionnables depuis l'extérieur du corps du patient, se caractérise en ce que lesdits moyens de fixation ou d'ancrage sont fixés auxdites deux extrémités par des moyens de connexion articulés et en ce que lesdits moyens de fixation ou d'ancrage sont en outre reliés l'un à l'autre par un élément de liaison rigide relativement parallèle à l'axe géométrique reliant lesdites deux extrémités de l'implant et situé à une certaine distance dudit axe, par des moyens de connexion également articulés, par exemple de façon à réaliser entre lesdits quatre points de connexion, un quadrilatère déformable, permettant un mouvement de rotation angulaire desdits moyens de fixation ou d'ancrage l'un par rapport à l'autre.

Ces moyens de connexion articulée peuvent être constitués d'articulations mécaniques proprement dites, ou de moyens de liaison convenablement déformables.

Les articulations peuvent être, par exemple, des articulations utilisant une rotule reçue de façon rotative dans un siège de la pièce d'extrémité, cette rotule présentant un passage à travers lequel elle peut recevoir et maintenir une partie d'un moyen d'ancrage telle qu'une vis pédiculaire. Bien entendu, tous les autres principes d'articulation telle qu'articulation à pivot peuvent être utilisés.

Dans une forme de réalisation, ledit élément de liaison est situé entre l'axe de l'implant et les éléments osseux auxquels l'implant est fixé, mais dans une autre forme de réalisation, ledit élément de liaison est disposé de l'autre côté de l'axe de l'implant par rapport aux éléments osseux réunis par l'implant.

Lesdits éléments de liaison peuvent être de simples liens rigides tels que des tiges ou barreaux de longueur invariable.

Cependant dans une autre forme de réalisation ces éléments de liaison peuvent eux-mêmes comporter, entre leurs extrémités, au moins une zone déformable, ce qui permet alors d'assurer des déplacements, tels que par exemple un allongement de l'implant sans rotation relative des moyens de fixation, par modification de longueur simultanée de l'élément proprement dit et de l'élément de liaison et/ou d'assurer un amortissement viscoélastique entre les éléments osseux sans mouvement de rotation desdits moyens de fixation.

On peut réaliser un implant complexe conformément à l'invention en utilisant deux implants individuels disposés, par exemple, côte à côte, par exemple de part et d'autres des apophyses épineuses du rachis, en assurant une interconnexion hydraulique permettant d'assurer l'une au moins des fonctions suivantes :
mouvement de rotation antisymétrique des moyens d'un implant individuel par rapport au mouvement des moyens d'ancrage de l'autre implant,
mouvements symétriques desdits moyens d'ancrage,
mouvements indépendants.

Bien entendu les deux éléments individuels d'un implant complexe peuvent, dans un but de simplicité, utiliser des éléments hydrauliques communs, tels que, par exemple, réservoir haute pression, collecteur basse pression, moyens d'établissement de la haute pression, moyens de commande de circuit hydraulique.

Dans une autre forme de réalisation, destinée à permettre une rotation dans un plan transversal ou éventuellement oblique, par rapport à la direction générale de l'implant, c'est-à-dire à la direction reliant les deux moyens ou vis d'ancrage, l'implant squelettique, présentant un premier et un deuxième élément d'extrémité, des moyens de fixation ou d'ancrage, dans des parties osseuses, par exemple par l'intermédiaire de vis, par exemple des vis pédiculaires situées aux extrémités, au moins un élément déformable, interposé entre les deux éléments d'extrémité, et ses moyens d'actionnement, pour permettre une modification durable, de préférence obtenue progressivement, de la distance entre une extrémité et un élément mobile par rapport à ladite une extrémité, et des moyens de commande, de préférence actionnables depuis l'extérieur du corps du patient, se caractérise en ce que, ledit élément mobile est agencé pour provoquer ou autoriser une rotation de l'autre des deux extrémités autour d'un axe sensiblement parallèle audit implant.

Les moyens par lesquels le déplacement dudit élément mobile par le mouvement de l'élément déformable, transforme ce mouvement en un mouvement de rotation de ladite autre extrémité et du moyen de fixation ou d'ancrage qu'elle supporte, peut-être un moyen associant une vis et un écrou de sorte que le mouvement de translation de l'un provoque un mouvement de rotation de l'autre.

Dans une forme de réalisation particulière, l'élément déformable est interposé entre les deux pièces d'extrémité dont l'une est susceptible de tourner, de sorte que la déformation de l'élément déformable entraîne à la fois une rotation et une translation de l'un des éléments d'extrémité, et donc des moyens d'ancrage qu'il porte, par rapport à l'autre pièce d'extrémité, se traduisant par un mouvement hélicoïdal de l'une des pièces d'extrémité par rapport à l'autre.

Dans une autre forme de réalisation, les deux pièces d'extrémité peuvent être solidaires l'une de l'autre de façon à rester écartées d'une distance invariable, l'élément mobile étant alors interposé entre cet ensemble d'éléments d'extrémité et le moyen de transformation de la déformation de l'élément mobile, en rotation de l'un des éléments d'extrémité par rapport à l'autre.

Dans une autre forme de réalisation, c'est l'élément déformable lui-même qui est construit pour se déformer en rotation, par exemple en utilisant une chambre déformable à piston rotatif, selon les principes bien connus de la rotation hydraulique.

Des dispositifs susceptibles de rotation, tels qu'ils viennent d'être décrits, peuvent être particulièrement utiles dans le cas des scolioses graves ou des déstablisations dégénératives graves du rachis. On pourra alors, par exemple, fixer deux dispositifs selon l'invention de part et d'autre de l'apophyse postérieure vertébrale, entre deux étages vertébraux, et provoquer la rotation entre les deux ancrages de l'un des deux dispositifs, ou une rotation associée à un déplacement longitudinal, l'autre dispositif étant susceptible alors d'un mouvement d'adaptation géométrique complémentaire des déplacements imposés par le premier.

Toutes sortes de déplacements complexes non rectilignes peuvent être obtenus, grâce à l'intervention, par exemple, en assujetissant le déplacement d'un moyen d'ancrage, ou d'une pièce d'extrémité, à une came ou glissière ou autre guide incurvé.

De préférence, l'implant constitué par un dispositif selon l'invention possédera une forme extérieure bien adaptée à l'environnement corporel dans lequel il se trouve. Par exemple, notamment pour des implants vertébraux, il sera avantageux de donner à chacune des deux extrémités une forme fuselée de façon à ne pas gêner les tissus voisins, d'autant plus que, grâce aux implants selon l'invention on peut aboutir à une amélioration fonctionnelle à laquelle devront être associés les muscles et ligaments, contrairement aux arthrodèses, qui se traduisent par leur atrophie.

Cette forme fuselée peut comprendre une enveloppe, de préférence déformable appliquée autour de l'implant, dont les deux extrémités, qui présentent les moyens de fixation pour les moyens d'ancrage, émergent de l'enveloppe, laquelle contient les différents autres constituants de l'implant. Le volume inteme libre dans cette enveloppe peut, de préférence, servir de réservoir de liquide basse pression. De préférence l'implant comporte à l'intérieur de cette enveloppe, l'élément mobile, qui peut être un moteur mécanique, ou, de préférence un moteur hydraulique, par exemple un soufflet hydraulique dont l'intérieur est relié, par une vanne basse pression, au volume basse pression formé dans l'enveloppe.

De préférence, à l'intérieur de l'enveloppe, on trouve également un réservoir haute pression, de préférence un soufflet, et, également de préférence, un élément déformable différentiel pour envoyer du liquide sous très haute pression dans le réservoir haute pression, le réservoir haute pression étant relié à l'élément mobile également par une vanne haute pression, les deux vannes haute pression et basse pression étant, de préférence, relativement éloignées l'une de l'autre, afin de permettre facilement une commande sélective par des moyens de commande externes, tels que, par exemple, des aimants.

Cependant, dans une autre forme de réalisation, notamment, lorsque l'on prévoit une succession d'implants individuels à divers étages du rachis, on peut également prévoir un réservoir haute pression unique et/ou un moyen de recharge unique des réservoirs hautes pressions, situé à l'écart des différents implants individuels et relié à ceux-ci par des conduits inextensibles.

Le réservoir de haute pression peut être avantageusement conçu pour maintenir le liquide, qu'il contient, sous haute pression, même lorsque des quantités notables de ce liquide sont envoyées aux moyens moteurs de l'implant, provoquant une diminution sensible du volume de liquide. Ceci peut être assuré, par exemple, en réalisant le réservoir haute pression sous forme de réservoir déformable élastique de grande raideur, par exemple un soufflet métallique de grande raideur et qui est expansé lorsqu'il contient le liquide sous haute pression, ce soufflet tendant à se rétracter pour maintenir la haute pression pendant une partie importante de sa course de rétraction. En variante, ou en association avec un tel soufflet, on peut également prévoir, pour maintenir la valeur élevée de pression dans le réservoir, un accumulateur d'énergie sous forme d'une enceinte à paroi élastiquement déformable qui est comprimée, donc diminuée de volume, lorsque le liquide sous haute pression est introduit dans le réservoir haute pression, et qui se détend élastiquement en maintenant une pression élevée lorsque du liquide est soutiré hors du réservoir haute pression. Cet accumulateur d'énergie est, de préférence, formé par une capsule déformable, qui peut, par exemple, comprendre à son intérieur une matière facilement déformable ou un gaz, mais qui de façon particulièrement préférée présente un vide important de façon à supprimer tout risque de fuite de gaz.

D'autres avantages et caractéristiques de l'invention apparaîtront à la lecture de la description suivante, faite à titre d'exemple non limitatif et se référant au dessin annexé dans lequel :
la figure 1 représente une vue schématique d'une paire d'implants pour des rotations antisymétriques selon une première forme de réalisation de l'invention,
la figure 2 représente une autre forme de réalisation d'une telle paire d'implants,
la figure 3 représente une autre forme de réalisation d'une telle paire d'implants,
la figure 4 représente une forme particulière de réalisation d'un implant de la figure 3,
la figure 5 représente une vue d'une paire d'implant selon l'invention pour des mouvements de rotation symétriques selon une première forme de réalisation,
la figure 6 représente une deuxième forme de réalisation d'une telle paire d'implants,
la figure 7 représente une troisième forme de réalisation d'une paire d'implants pour des rotations symétriques,
la figure 8 représente une vue d'une forme de réalisation d'un implant de la figure 7.

- la figure 9 représente une vue schématique d'une paire d'implants dont l'un permet un mouvement simultané axial et de rotation dans un plan transversal à la direction générale de l'implant.
- la figure 10 représente une vue schématique d'une paire d'implant dont l'un permet une rotation sans mouvement de translation, dans un plan perpendiculaire à la direction générale de l'implant,
la figure 11 représente une vue de côté d'une forme de réalisation d'un implant selon la figure 9.
- la figure 12 représente une vue en élévation avec section partielle de cet implant

la figure 13 représente une vue en élévation avec section partielle d'un implant selon une forme de réalisation de la figure 10.
la figure 14 représente en coupe, une forme de réalisation détaillée de l'invention

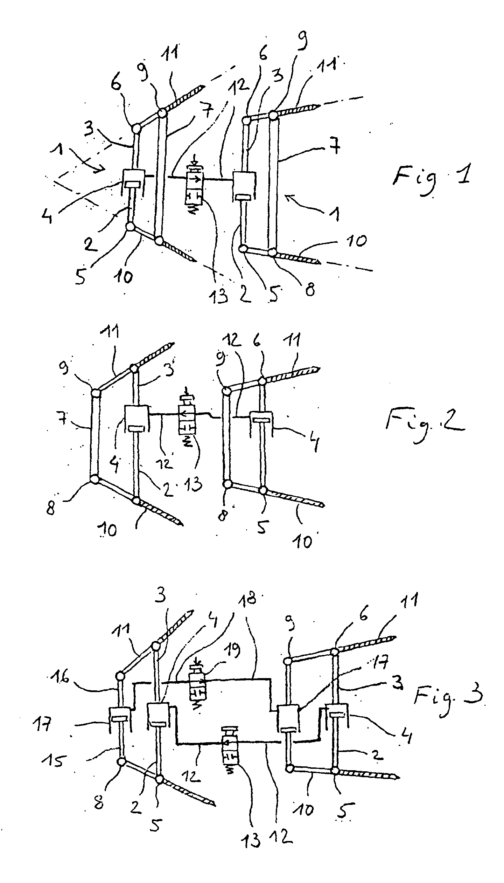

Sur les différentes figures 1 à 10 qui suivent, on suppose que le rachis s'étend dans une direction verticale et que les deux implants individuels sont disposés de part et d'autres de la succession des apophyses épineuses et que les vis pédiculaires inférieures sont vissées dans une première vertèbre et les vis pédiculaires supérieures dans une autre vertèbre, adjacente ou non, disposée au-dessus de la première. Les deux éléments individuels sont représentés dans un plan frontal, qui est le plan de la feuille du dessin. Il en résulte que les vis pédiculaires devraient être orientés dans un plan plus ou moins sagittal, donc plus ou moins perpendiculaire au plan de la feuille de dessin ou en tout cas incliné mais, pour des raisons de simplicité de représentation on les a représentées dans le même plan frontal. De même les éléments de liaison ont été représentés dans le même plan frontal alors qu'ils devraient être dans le plan perpendiculaire ou incliné qui contient les vis pédiculaires.

Sur la figure 1 on a représenté deux implants individuels droit et gauche généralement désignés par 1 et comprenant une tige d'extrémité inférieure 2 et une tige d'extrémité supérieure 3 entre lesquelles est interposé un élément hydraulique déformable 4 qui a été représenté sous forme d'un ensemble cylindre-piston mais qui dans la réalité serait plutôt réalisé sous forme d'un soufflet métallique de façon à éviter les fuites de liquide hydraulique. En variante, cet élément déformable peut être du type télescopique ou autrement déformable, par exemple une enceinte cylindrique élastique longitudinalement, mais non transversalement. Les tiges 2 et 3 sont guidées dans le prolongement l'une de l'autre pour se déplacer le long du même axe vertical et adopter des positions écartées ou rapprochées en fonction du plus ou moins grand remplissage de l'élément déformable 4. Les extrémités inférieure et supérieure 2, 3 présentent des moyens de connexion 5, 6 sous forme d'articulations. Parallèlement à l'élément 1, s'étend une tige rigide de liaison 7 se terminant par des moyens de connexion inférieurs 8 et supérieurs 9 sous forme d'articulations. La tige 7 s'étend sensiblement parallèlement à l'élément individuel 1, mais pourrait être plus inclinée, et peut être rendue solidaire de cet élément, sans que cela soit toutefois une nécessité. A chaque élément 1 est associé une vis pédiculaire inférieure 10 et une vis pédiculaire supérieure 11 dont les parties filetées sont fixées dans les pédicules vertébraux correspondants. L'extrémité postérieure des vis pédiculaires 10, 11 est reçue dans les extrémités 5 et 6 à la façon d'une articulation autorisant un débattement angulaire au moins dans le plan constitué par l'élément 1 et l'élément de liaison 7. Eventuellement, l'articulation peut avoir un degré de liberté supplémentaire ou être réalisée sous forme sphérique donnant un degré de liberté en rotation dans toutes les directions.

Dans un emplacement intermédiaire les vis 10, 11 se trouvent fixées et articulées respectivement aux extrémités 8 et 9, de l'élément de liaison 7 par des articulations autorisant également un débattement angulaire dans le plan commun, par exemple sagittal, de l'élément 1 et de son élément de liaison 7.

Les deux éléments déformables 4 de la paire d'implants individuels qui forment l'implant complexe représenté sur le dessin, sont reliés par une canalisation 12 sur laquelle est disposé un élément de circuit hydraulique 13 représenté, dans l'exemple, sous forme d'une vanne-tiroir.

Le montage représenté sur la figure 1 permet des mouvements de rotation antisymétrique des vis pédiculaires.

On suppose que les vis pédiculaires ont été vissées dans les positions angulaires représentées sur le dessin, que les circuits hydrauliques et les éléments 4 sont entièrement remplis de liquide hydraulique et que la vanne 13 est en position fermée. Dans une telle situation les vis pédiculaires sont bloquées dans leur position angulaire représentée sur le dessin. Dans cette position le volume interne de l'élément 4 de gauche est inférieur à celui de l'élément 4 de droite qui correspond à un angle plus fermé. Si maintenant l'on ouvre la vanne, on se rend compte que les vis pédiculaires vont pouvoir pivoter autour du centre d'articulation des points 8 et 9 à l'extrémité de la tige de liaison, en fonction de l'accroissement ou de la diminution de longueur de l'élément 1 correspondant, elle-même dictée par le volume de liquide présent dans l'élément déformable associé 4. En raison de la communication 12 entre les deux éléments déformables 4 on conçoit également que toute variation de volume de liquide de l'un des éléments est compensée par une variation de volume en sens inverse dans l'autre de sorte que la rotation des vis pédiculaires dans un sens sur l'un des éléments se traduits par une rotation des vis pédiculaires dans l'autre sens et ayant sensiblement la même valeur angulaire absolue.

Cette propriété peut être mise à profit dans diverses applications décrites dans les demandes EP et US susmentionnées.

Si l'élément de circuit hydraulique 13 est un élément de réglage viscoélastique freinant fortement le passage de liquide, ou l'interdisant en cas de mouvement angulaire brusque des vis pédiculaires, on peut laisser les vertèbres libres de pivoter l'une par rapport à l'autre dans le plan frontal du rachis lorsque les mouvements sont lents et au contraire immobiliser les vis ou freiner fortement leur rotation lorsque les mouvements ont tendance à être rapides. On peut ainsi obtenir un effet d'amortissement en rotation tout en autorisant une liberté de rotation pour des mouvements lents.

Si l'élément 13 est un élément permettant d'imposer l'envoi du liquide hydraulique dans l'un des éléments déformables 4 et la soustraction du même volume de liquide dans l'autre élément 4, cet envoi étant suivi ensuite d'une fermeture de la communication, on peut, après avoir implanté les deux implants individuels selon des angles de vis pédiculaires données, provoquer, au bout d'un certain temps, par commande extérieure, par exemple par commande magnétique transcutanée, une modification de l'angle et provoquer ainsi, par petits paliers, un redressement d'une déformation vertébrale.

On peut prévoir des moyens permettant d'exercer une pression constante continue ou intermittente dans le soufflet 4, de façon à solliciter en permanence les parties squelettiques dont la position doit être corrigée.

Bien entendu, on peut grâce à des circuits hydrauliques différents, assurer les deux fonctions qui viennent d'être décrites, comme cela est explicité dans les demandes susmentionnées.

Bien entendu, conformément à l'invention, on peut également utiliser chaque implant individuel avec sa tige de liaison comme un élément totalement indépendant et commander séparément chacun des éléments sans aucune interconnexion 12 afin d'assurer certaines ou toutes les fonctions de modification de longueur et donc d'angulation, ainsi que d'amortissement viscoélastique.

De façon préférée l'élément d'implant est également associé à un dispositif permettant d'alimenter un élément déformable 4 en liquide haute pression, si cela est nécessaire, à partir d'un soufflet déformable fonctionnant par exemple comme une pompe actionnée par le corps, comme décrit par la susdite demande. Dans le cas de l'utilisation de deux implants individuels pour former un implant complexe comme représenté, ce moyen d'alimentation et de décharge peut être commun aux deux implants.

Sur la figure 2 on a représenté un implant complexe similaire à celui de la revendication 1 mais dans lequel les tiges de liaison 7 sont articulées aux extrémités libres ou têtes des vis pédiculaires alors que l'implant individuel déformable est articulé en position intermédiaire, ce qui donne une inversion du mouvement de rotation par rapport à celui représenté sur la figure 1, et éloigne en outre vers l'arrière, le centre de rotation de chaque vis pédiculaire.

Sur la figure 3 on a représenté un implant complexe identique, pour les implants individuels 1, à celui représenté sur la figure 2. Par contre l'élément de liaison 7 qui était une simple tige rigide, est remplacé par un élément de liaison 14 formé à la façon d'un implant individuel et comprenant donc deux extrémités 15, 16 susceptibles de se déplacer longitudinalement l'une par rapport à l'autre avec interposition d'un élément hydraulique déformable 17, ce qui permet d'avoir un élément de liaison dont on peut modifier la longueur si on le désire ou qui peut présenter lui-même un effet d'amortissement analogue à celui de l'implant 1 proprement dit si cette fonction est présente.

De préférence, les deux éléments 17 des deux implants individuels représentés sont reliés par une canalisation 18 avec interposition d'un élément 19 de circuit hydraulique.

Les fonctions recherchées seront alors déterminées par la nature et la commande des éléments de régulation hydraulique 13 et 19 et l'on conçoit, que dans une telle réalisation, on peut, si on le souhaite, rendre interchangeable l'élément d'implant 1 et son élément de liaison 14 et ainsi fixer le centre de rotation de la vis pédiculaire soit à l'extrémité 5 (ou 6), soit à l'extrémité 8 (ou 9), soit même en un autre point entre ces articulations.

Sur la figure 4 on a représenté schématiquement une forme de réalisation pratique du dispositif de la figure 3 (sur laquelle on n'a pas représenté les canalisations et les éléments de circuits hydrauliques 13, 17). L'implant individuel représenté comporte un soufflet hydraulique 4 portant à sa face supérieure une pièce avec un bras formant la tige 3, à sa face inférieure un plateau avec un bras 2 formant la tige inférieure, lesdites tiges présentant des articulations 5 et 6 pour les vis pédiculaires 10, 11. L'élément 14 comporte un soufflet hydraulique 17 dont le plateau inférieur porte un bras 15 et l'extrémité supérieure un bras 16, lesdits bas portant, à leur extrémité libre, les articulations 8, 9 recevant les extrémités postérieures des vis 10, 11. On peut éventuellement rendre mécaniquement solidaire un bras de l'élément 1 et un autre bras de l'élément 14 ou au contraire laisser tous ces éléments indépendants, la liaison ne s'effectuant que par les vis 10, 11.

On peut ainsi disposer spatialement les soufflets l'un sous l'autre et réaliser un implant selon l'invention avec un encombrement extrêmement réduit.

Sur la figure 5 on a maintenant représenté un dispositif analogue à celui de la figure 1 avec la seule différence que l'un des dispositifs déformables ou soufflets 4 a été remplacé par un dispositif déformable 20, qui peut d'ailleurs également être réalisé sous forme d'un soufflet, et dans lequel les points de fixation des bras inférieur 2 et supérieur 3 ont été inversés, de sorte qu'une augmentation du volume du dispositif 20 entraîne, contrairement à l'augmentation de volume du dispositif 4, un raccourcissement de l'élément d'implant au lieu d'un allongement.

Il est ainsi possible, grâce à l'interconnexion par le conduit 12 et l'élément 13 d'obtenir non pas des mouvements de rotation antisymétrique mais des mouvements de rotation symétriques. En d'autres termes les rotations des vis 10, 1 du côté droit du rachis sont identiques aux rotations des vis de l'élément gauche, et de même sens.

On peut ainsi autoriser des mouvements de flexion ou d'extension du rachis cette fois dans le plan sagittal.

Comme dans les autres cas, ceci peut être mis en oeuvre soit pour provoquer un effet de lordose ou inverse, selon le but recherché, par exemple en agissant par paliers à partir d'une commande externe, soit pour réaliser un effet d'amortissement parfaitement symétrique en cas de mouvement spontané de rotation entre les vertèbres, soit encore pour assurer simultanément les deux fonctions grâce à des circuits plus complexes.

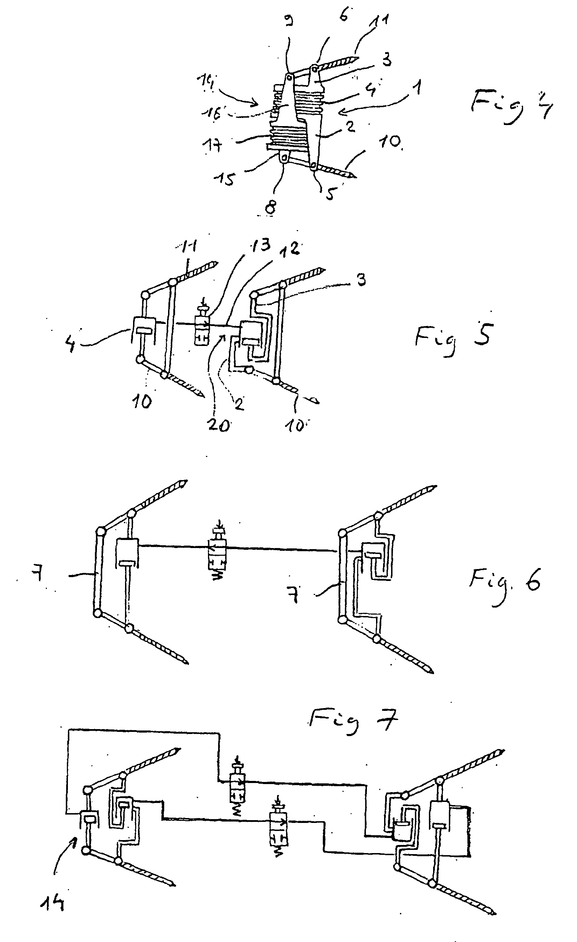

Sur la figure 6 on a représenté un montage selon la figure 5 mais dans lequel les éléments de liaison 7 sont disposés, comme sur la figure 2, de façon que le centre de rotation des vis pédiculaires soit disposé aux extrémités même des vis.

On peut également, en variante, associer la solution de la figure 1 et de la figure 2, en mettant, par exemple à droite du rachis, 1, l'élément selon la figure 1, et à gauche un élément individuel selon la figure 2, étant entendu que dans ce cas, les variations angulaires des vis de droite seront toujours en sens inverse de celles des vis de gauche mais de valeur absolue plus différente.

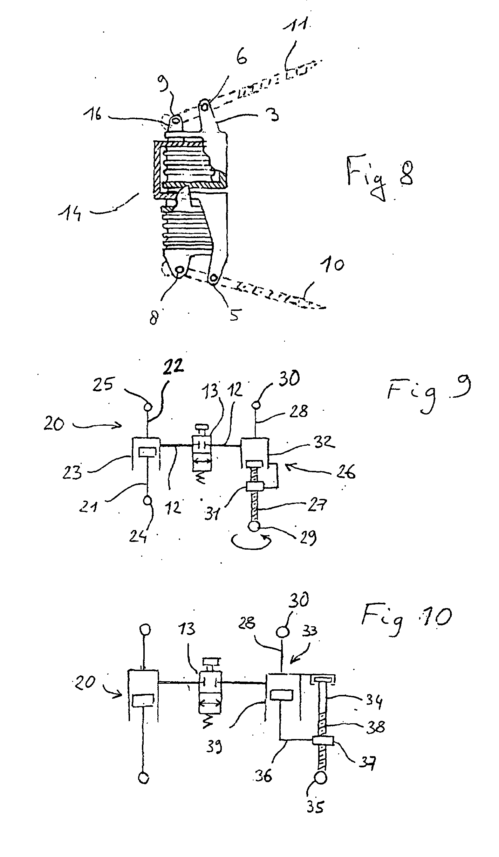

Sur la figure 7 on a représenté un implant complexe permettant également des relations symétriques dans le plan sagittal comme indiqué sur la figure 6 mais dans lequel la tige de liaison 7 de chacun des implants individuels a été remplacé par des éléments de liaison eux-mêmes de longueur variable, celui de gauche étant un élément de liaison 14 comme représenté sur la figure 3 alors que l'élément de liaison de droite possède également une inversion d'action au niveau du soufflet. En d'autres termes on obtient une disposition dans laquelle les mouvements de rotation de gauche et de droite sont symétriques dans le plan sagittal.

Sur la figure 8 on a représenté schématiquement une forme de réalisation analogue à la figure 4 mais dans laquelle on voit qu'en inversant les extrémités de soufflets sur lesquels les bras sont fixés, on obtient un mouvement de sens contraire à celui de la figure 4.

En se référant à la figure 9, on voit un ensemble de deux implants individuels dont l'implant de gauche, 20 comporte deux pièces d'extrémité 21, 22, susceptibles de se déplacer dans le prolongement l'une de l'autre avec interposition d'un élément déformable 23 analogue à l'élément déformable 4. Les extrémités 24 et 25 des pièces 21, 22 présentent des trous de fixation permettant la solidarisation de moyens d'ancrage, par exemple, des vis pédiculaires. Contrairement aux représentations des figures précédentes, ces moyens de fixation des extrémités 24 et 25 ne permettent pas obligatoirement un pivotement des vis pédiculaires, telles que les vis 10 et 11, et peuvent au contraire être réalisés par des perçages ou oeillets permettant une solidarisation rigide sans possibilité de pivotement de la vis par rapport à son extrémité correspondante 24 ou 25.

L'implant 26 de droite présente également deux pièces d'extrémité disposées dans le prolongement l'une de l'autre, à savoir 27 et 28, dont les extrémités 29 et 30 sont analogues aux extrémités 24 et 25 pour recevoir les vis pédiculaire sans possibilité de mouvement de la vis par rapport à l'extrémité qui la porte. La pièce d'extrémité 27 présente, depuis l'extrémité 29, une partie en forme de tige filetée allongée aboutissant à la partie mobile d'un élément hydraulique déformable 32 analogue à l'élément 23 ou à l'élément 4. On comprend donc que si l'élément déformable 32 se déforme et provoque un mouvement relatif, par exemple d'écartement ou distraction entre les pièces 27 et 28, le mouvement de la pièce 27 par rapport à la pièce 28 va provoquer la rotation de la pièce 27 du fait que sa tige filetée se déplace dans l'écrou fixe 31. Il en résulte que l'extrémité 29 est animée par rapport à l'extrémité 30 d'un mouvement de déplacement à la fois en translation et en rotation. En conséquence, l'extrémité de la vis pédiculaire (non représentée) portée par l'extrémité 29 de la pièce mobile 27 va décrire un mouvement hélicoïdal dont l'axe est constitué par l'alignement des pièces 27 et 28.

Si l'on relie les deux éléments 20 et 26 comme représentés sur la figure, par une vanne 13 dans un conduit 12, on comprend que, comme sur la figure 1, la diminution de volume de l'élément mobile 23 se traduira par une augmentation du volume de l'élément mobile 32 et donc des déplacements axiaux opposés des implants 20 et 26, avec en plus, le mouvement de rotation de la pièce 27.

On se réfère maintenant à la figure 10. Sur cette figure, l'élément 20 est identique à celui de la figure 9. Comme l'implant 26, l'autre implant 33 possède une pièce d'extrémité 28 se terminant par une extrémité 30 permettant la fixation et le blocage d'une vis d'ancrage. Par contre, l'élément 33 comporte une deuxième pièce d'extrémité 34 avec son extrémité 35 pour la réception et le blocage des vis d'ancrage, cette pièce 34 étant reliée à l'élément 28 de façon à être immobilisée en translation mais libre en rotation autour de l'axe de la pièce 34. La partie mobile 36 de l'élément déformable 39 présente une pièce en forme d'écrou taraudée 37 à travers laquelle passe une partie filetée 38 de la pièce 34. On comprend donc que lorsque l'élément déformable 39 se déforme, le mouvement de la pièce mobile 36 va provoquer une rotation de la pièce d'extrémité 34 autour de son axe mais sans translation par rapport à la pièce d'extrémité 28, de sorte que l'extrémité 35 tourne sans déplacement en translation par rapport à l'extrémité 30.

Dans la forme de réalisation représentée sur la figure 10, grâce à vanne 13, une variation de volume de l'élément déformable 20 va se traduire par une variation de volume inverse de l'élément déformable 39, de sorte que l'allongement de l'élément 20 se traduit par une rotation dans un sens de l'extrémité 35 alors que le raccourcissement de l'élément 20 produit une rotation en sens inverse de l'extrémité 35.

Bien entendu, toutes les autres combinaisons de commande peuvent être réalisées, par exemple dans le cas de la figure 9 pour provoquer des allongements identiques des éléments 20 et 26 tout en assurant la rotation de la pièce 27.

En se référant aux figures 11 et 12, on a représenté des formes de réalisation de l'implant 26 de la figure 9. On voit que les pièces d'extrémité 27 et 28 ont une forme fuselée en nez de dauphin et présentent des passages transversaux formant les extrémités 29 et 30 et permettant, d'une façon tout à fait classique, la fixation d'une vis pédiculaire. Sur cette figure, la portion formant écrou 31 est portée par la pièce inférieure 27. Cette écrou 31 est traversée par une tige filetée 40 rigidement portée par la pièce d'extrémité supérieure 28 de sorte que le déplacement axial de cette tige provoque la rotation de la pièce 28 par rapport à la pièce 27. La tige filetée 40, comme on le voit bien sur la figure 12, pénètre dans un pot 41 reçu de façon étanche à l'intérieur du soufflet hydraulique 32 jouant le rôle d'élément mobile et la tige 40 peut tourner dans ce pot autour de son axe en étant retenue à l'intérieur du pot par un circlips 42. On en voit qu'on peut en plus donner à l'ensemble de l'implant une forme fuselée allongée particulièrement impropriée à une bonne cohabitation avec le tissu environnant.

En se référant à la figure 13, on voit une forme de réalisation d'un implant 33 selon la figure 10 avec une extrémité supérieure 28 et une pièce d'extrémité inférieure 34 qui présente des bras 43 aboutissant à un palier 44 à l'intérieur duquel peut librement tourner, en étant retenue axiellement par un circlips 45 une tige filetée 46 solidaire de la pièce 28 et susceptible de tourner à l'intérieur d'un écrou 47 que présente un pot 48 fixé de façon étanche sur le soufflet métallique 49 mollo extrémité solidaire de la pièce d'extrémité 34. On comprend que tout mouvement du soufflet provoque un mouvement axial de l'écrou 47, ce qui provoque une rotation sans mouvement axial de la pièce 28 par rapport à la pièce 34.

On va maintenant décrire la mise en oeuvre d'un implant double pour la correction d'une scoliose chez un patient ayant, entre les deux étages vertébraux scoliotiques, un angle de scoliose a1. Pendant l'opération, le chirurgien par des moyens classiques, réalise une pré-correction ramenant l'angle de scoliose à la valeur a2 < a1. Il pose ensuite les deux implants individuels de part et d'autre de la colonne vertébrale entre les deux étages vertébraux considérés, la vanne 13 étant ouverte, ce qui permet le raccourcissement de l'un des implants individuels et l'allongement compensateur de l'autre élément individuel et il fixe les deux éléments à l'aide de leur vis pédiculaire. Il suffit ensuite de refermer la vanne 13 de sorte que les deux implants individuels se trouvent bloqués dans leur position sans possibilité de mouvement et maintiennent la partie scoliotique de la colonne vertébrale dans l'angle a2. A partir de ce moment, toutes les charges sont portées par la prothèse constituée par l'implant double.

Après un délai raisonnable postopératoire, pendant laquelle les efforts sont supportés essentiellement par la prothèse, le point neutre de la colonne vertébrale se sera adapté grâce à la réorganisation des tissus squelettiques et para- vertébraux, ce qui diminuera la charge de la prothèse à la station debout. On peut soit estimer cette diminution de charge, soit prévoir dans la prothèse des capteurs de pression interrogeables, de préférence, de façon non invasive comme cela est déjà bien connu et qui indiqueront que la colonne vertébrale est arrivée à un état d'équilibre.

On procédera alors à un nouveau réglage en demandant au patient de s'incliner du côté où il fait diminuer la valeur de l'angle a de la scoliose à une valeur a2 < a1, après avoir ouvert la vanne 13, ce qui rend les deux implants mobiles. Une fois cet angle a2 obtenu, on referme la vanne de sorte que les deux implants maintiennent cette nouvelle position et empêchent le retour à un angle de scoliose plus grand. Ce blocage de la prothèse se traduit chez le patient par la perception d'une sensation d'obstacle qui disparaîtra progressivement jusqu'à l'obtention d'un nouvel équilibre.

Par une succession de ces manoeuvres, il est ainsi possible de réduire voire même de supprimer l'angle de scoliose et de retirer la prothèse.

On comprend que les mêmes principes peuvent être utilisés pour le rétablissement progressif d'une cyphose ou d'une lordose en utilisant des paires d'implants agencées par exemple selon les figures.

De même, en utilisant par exemple d'une paire de prothèse selon la figure 9, il serait possible de procéder progressivement à la réduction d'une cypho-scoliose.

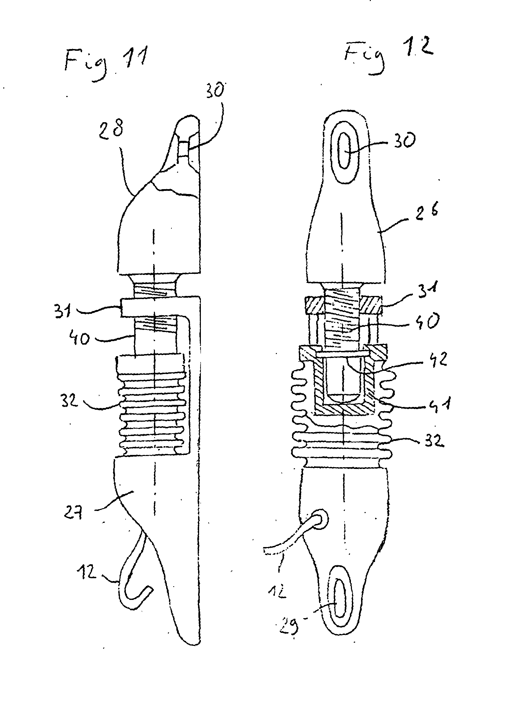

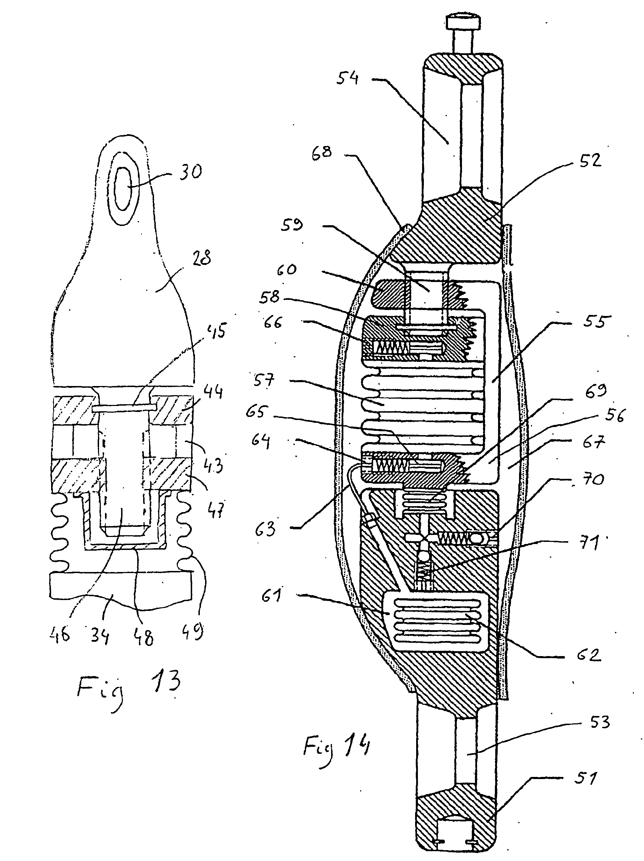

On a représenté, sur la figure 14, une vue en coupe transversale d'un implant sous une forme de réalisation perfectionnée de l'invention.

Cet implant 1 comporte deux pièces d'extrémités 51 et 52 en forme de tête de dauphin, présentant à leur partie la plus extrême, des trous ou oeillets 53 et 54 qui peuvent être agencés pour être traversés par des vis pédiculaires dont les têtes peuvent être solidement fixées au niveau des trous de 53, 54. En variant, ces trous peuvent être agencés de façon à permettre une articulation de la tête de la vis pédiculaire et donc un déplacement angulaire entre l'élément d'extrémité et la vis qu'il porte.
Entre les deux pièces d'extrémités 51 et 52 est disposée une troisième pièce 55 mobile par rapport aux deux extrémités. La pièce 55 présente une forme d'étrier dont l'une des branches, 56, supporte de façon étanche un soufflet métallique 57 dont l'extrémité libre est fixée, de façon étanche, contre une pièce 58 susceptible de coulisser par rapport à l'étrier 55 et portant, de façon tourillonnante, grâce à un circlips, mais axialement non mobile par rapport à la pièce 58, une tige filetée 59 qui traverse un taraudage complémentaire de la seconde branche 60 de la pièce 55. On comprend donc que, lorsque l'élément déformable constitué par le soufflet 57 se déforme, le déplacement axial ainsi provoqué de la tige 59 solidaire de l'extrémité supérieure 3 entraîne la rotation de cette tige dans l'écrou fixe formé dans la branche 60, et par conséquent, un mouvement simultané de translation et de rotation de la pièce d'extrémité 52 par rapport à la pièce 60.

A l'intérieur de la pièce d'extrémité 51 est disposée une cavité étanche 61 servant de chambre haute pression et dans laquelle se trouve un soufflet 62 hermétiquement scellé et dans lequel on a fait le vide, la raideur de ce soufflet étant cependant suffisante pour qu'il tende spontanément à se déployer et augmenter de volume même lorsqu'il est environné par la haute pression régnant dans la chambre 61. La chambre 61 communique, par un conduit indéformable 63, avec l'intérieur du soufflet métallique 57 par l'intermédiaire d'une vanne haute pression 64 logée dans la branche 56, cette vanne possédant un tiroir tubulaire en fer doux 65 normalement repoussé par un ressort dans la position obturant le passage vers le soufflet 57. On comprend donc que lorsque l'on amène le noyau plongeur 65 dans une position d'ouverture à l'encontre du ressort de vanne, du liquide se trouvant à haute pression dans la chambre 61 va parcourir le conduit 63 et pénétrer dans le soufflet 57, le maintien de la haute pression de la chambre 61 étant assuré par la déformation concomitante du soufflet scellé 62. Cet afflux de liquide provoque le déplacement de la pièce 58 vers la branche 60 et, par conséquent, la distraction et la rotation de la pièce d'extrémité 52 par rapport à la pièce centrale 55.

L'intérieur du soufflet 57 communique, par ailleurs, par l'intermédiaire d'une vanne basse pression 66 munie d'un noyau plongeur identique au noyau 65, située dans la pièce 58, avec le volume 67, entourant les différentes pièces contenues à l'intérieur du manchon déformable, imperméable 68, aux deux extrémités duquel émergent les extrémités des pièces 51 et 52, ce volume 67 formant le volume basse pression. On comprend donc que lorsqu'on ouvre la vanne 66, du liquide contenu dans le soufflet 57 va sortir et se répandre dans le volume basse pression 67, permettant alors une rétraction ou compression du soufflet 57 et une rotation simultanée en sens inverse de la pièce 52.

La recharge du réservoir haute pression 61 s'effectue par l'intermédiaire d'un soufflet métallique 69, de diamètre nettement plus faible que celui du soufflet 57 et qui est interposé entre les pièces 51 et 55. Ce soufflet 69, lorsque les pièces 51 et 55 s'éloignent, s'expanse et aspire du liquide depuis la chambre basse pression 67 par l'intermédiaire d'un clapet de non retour 70. Au contraire lorsque les pièces 51 et 56 se rapprochent, la forte pression engendrée dans le soufflet 69 fait pénétrer du liquide, sous très haute pression, dans la chambre haute pression 61 par l'intermédiaire d'un clapet de non retour 71.

Il n'est pas nécessaire que la déformation du soufflet 69 soit de grande amplitude ; au contraire, on préfère que l'intervalle entre la pièce 51 et la pièce 56 soit faible et que, la course d'oscillation entre les pièces 51 et 55 soit limitée, une multitude d'oscillations, par exemple lors de la marche du sujet ou de changements de position corporelle suffisant à générer la haute pression permettant d'alimenter la chambre 61.

Dans une telle forme de réalisation, tant qu'aucune des vannes 64 et 66 n'est ouverte, les deux pièces 51 et 52 ne peuvent se déplacer, l'une par rapport à l'autre, que d'une très faible course, et elle assure donc le maintien dans la position choisie des deux éléments squelettiques auxquels elles sont ancrées, par exemple deux vertèbres. On peut même mettre à profit le dispositif d'établissement de haute pression pour réaliser un certain amortissement visqueux des petits déplacements autorisés entre les pièces 51 et 55.

On comprend, par ailleurs, qu'en ayant disposé les vannes haute pression et basse pression 64, 66 de part et d'autre du soufflet 67, on peut facilement actionner, au choix, l'une ou l'autre de ces vannes, par exemple, au moyen d'un fort aimant placé sur la peau au regard de l'une des vannes pour attirer le plongeur ferromagnétique tel que 65 vers la gauche du dessin et ouvrir la vanne.

On comprend, bien entendu, que l'on pourrait réaliser un implant analogue à celui qui vient d'être décrit, mais agencé pour ne pas provoquer de rotation entre les deux pièces d'extrémités, mais simplement un mouvement de distraction ou de compression. On pourrait également réaliser un implant tel que celui de la figure 13 utilisant la plupart des dispositions constructives de la figure 14 pour réaliser un implant uniquement rotatif.

En associant à un implant de ce genre une tige de liaison analogue aux tiges 7 et en réalisant, aux extrémités des pièces 51 et 52, des portées d'articulations autorisant un pivotement des vis pédiculaires par rapport audites extrémités, on peut également réaliser des implants selon les figures 1,2, ou 5, ou 6.

Dans le cas où l'on utilise un grand nombre d'implants selon l'invention disposés le long de la colonne vertébrale entre divers étages rachidiens, on peut également prévoir un réservoir haute pression et un réservoir basse pression uniques ainsi qu'un élément déformable d'établissement de haute pression unique, cet ensemble de réservoir étant disposé à l'écart des différents implants individuels et étant relié à chacun de ceux-ci par une conduite basse pression et une conduite haute pression, les implants ne possédant alors eux-mêmes aucun élément déformable hydraulique autre que le soufflet moteur jouant le rôle du soufflet 57.

Egalement les vannes commandables, telles que les vannes 13, 19 ou 64 ou 66, au lieu d'être commandées directement par l'intermédiaire d'un plongeur ferromagnétique susceptible d'être attiré par un aimant placé à la surface de la peau au voisinage de la vanne, pourraient être commandées, d'une façon en soi connue en hydraulique, par une petite vanne pilote, plus facile à actionner car ayant un plongeur d'une plus faible inertie, la vanne pilote adressant une pression de commande à la vanne d'interruption proprement dite pour ouvrir celle-ci, la fermeture de la vanne pilote provoquant au contraire la fermeture de la vanne principale.

On comprend, par ailleurs, qu'on peut limiter le mouvement de l'une des extrémités par rapport à l'autre en prévoyant des moyens de butée classique entre les deux pièces, qui viennent s'appliquer si la course de l'organe déformable ou du soufflet moteur dépasse une amplitude désirée. On obtient ainsi une sécurité, dans le cas d'un défaut de fonctionnement de l'implant qui l'empêche de maintenir exactement la position souhaitée, par exemple, fuite de liquide ou déformation de conduite ou déformation excessive d'un soufflet.

De préférence, les implants, selon l'invention, sont livrés avec un élément provisoire amovible qui les maintient dans une position d'écartement désirée entre les deux éléments d'extrémités et que le chirurgien dépose, une fois qu'il a implanté l'implant et fixé les vis pédiculaires ou autres moyens d'ancrage aux extrémités de l'implant.

L'invention a également pour objet un procédé chirurgical thérapeutique consistant à assurer une modification de la position de deux portions ou éléments du squelette, par exemple, deux vertèbres, dans lequel, on implante au moins un élément d'implant selon l'invention, on fixe l'une des extrémités par un moyen d'ancrage à l'une des portions ou éléments squelettiques, et l'autre extrémité par un moyen d'ancrage à l'autre portion ou élément squelettique, après avoir, éventuellement, procédé à une première correction de la position relative desdites deux portions ou éléments, on laisse cicatriser la voie d'abord et les tissus sur lesquels on est intervenu, puis on commande, de préférence par un moyen de commande non invasif, un déplacement ou une force de correction en sollicitant de façon correspondante les deux pièces d'extrémités de l'implant, ou les moyens d'ancrage, l'un par rapport à l'autre.

Ce déplacement peut être provoqué directement par le corps du patient et, dans ce cas, on autorise le déplacement par une autorisation de déformation de l'élément mobile, par exemple un soufflet hydraulique, puis le déplacement étant achevé, on interdit tous les déplacements ultérieurs par blocage dudit élément déformable.

Dans une autre forme de réalisation, pour provoquer le déplacement, on provoque temporairement une déformation de l'élément mobile en appliquant une force permettant d'obtenir le déplacement désiré, après quoi on rebloque et on empêche le mouvement de l'élément déformable.

Dans une troisième forme de réalisation au contraire, on permet à l'élément mobile d'exercer une force permanente, de préférence constante ou éventuellement progressivement variable, entre les deux extrémités et donc les deux portions ou éléments squelettiques avec une intensité de force insuffisante pour provoquer une modification brutale de dimension et une atteinte au tissu s'opposant à cette variation dimensionnelle, mais suffisante pour provoquer, comme cela étant soi connu dans le domaine chirurgical, une lente déformation et l'adaptation des différents tissus jusqu'à atteindre la position corrigée souhaitée.

Un tel procédé est particulièrement applicable à la correction des scolioses ou des cyphoscolioses.

Lorsque l'on exerce une force, grâce à un implant selon l'invention, entre deux éléments squelettiques, cette force peut être avantageusement comprise entre quelques daN à 25 ou 30 daN.

Le dispositif peut avantageusement comporter des capteurs de force ou de pression pour limiter ou régler la force à exercer. De tels capteurs miniaturisés sont disponibles dans le commerce.

On a vu que les moyens de commande non invasifs peuvent être des aimants qui, de l'extérieur du corps, peuvent déplacer ou attirer une masse ferromagnétique, tel qu'un tiroir de vanne, à l'encontre d'un ressort ou moyen de rappel élastique, qui ramène la masse à sa position initiale, une fois l'aimant éloigné.

On peut aussi utiliser un dispositif externe qui crée un champ magnétique ou électromagnétique rotatif qui, à l'intérieur du corps, fait tourner une pièce rotative, par exemple un tiroir rotatif de vanne.

## Revendications

1. Implant squelettique, présentant un premier et un second élément d'extrémité (2, 3, 15, 16, 21, 22, 27, 28, 34, 38 51, 52) des moyens d'ancrage (10,11) susceptibles d'être fixés dans des parties osseuses, reliés respectivement auxdits premier et second éléments d'extrémité, au moins un élément déformable (4, 17, 23, 32, 39, 57) relié respectivement auxdits premier et second éléments d'extrémité et autorisant un mouvement entre eux, et donc entre lesdits moyens d'ancrage, **caractérisé en ce qu'**il comporte des moyens d'articulation (5, 6, 8, 9, 24, 25, 31, 37, 60) autorisant une rotation desdits moyens d'ancrage autour d'un axe perpendiculaire et/ou d'un axe parallèle à la direction géométrique rejoignant lesdits éléments d'extrémité, et **en ce qu'**il comporte un moyen moteur dudit élément déformable pour provoquer sa déformation, entraînant ladite rotation.

2. Implant selon la revendication 1, **caractérisé en ce que** ledit moyen moteur est un moyen hydraulique.

3. Implant selon la revendication 1 et 2, **caractérisé en ce que** ledit élément déformable est déformable en rotation.

4. Implant selon la revendication 1, **caractérisé en ce que** ledit organe déformable est un soufflet alimenté à partir d'un réservoir hydraulique.

5. Implant squelettique selon l'une des revendications 1, 2 et 4, **caractérisé en ce que** lesdits moyens d'ancrage (10, 11) sont reliés auxdites deux extrémités (2, 3) par des moyens de connexion articulés (5, 6) et **en ce que** lesdits moyens d'ancrage (10, 11) sont en outre reliés l'un à l'autre par un élément de liaison (7, 14) situé à une certaine distance desdits éléments d'extrémité, et portant des moyens de connexion supplémentaires (8, 9) également articulés permettant un mouvement angulaire de rotation desdits moyens d'ancrage (10, 11) l'un par rapport à l'autre.

6. Implant selon la revendication 5, **caractérisé en ce que** ledit élément (7, 14) de liaison est situé de l'autre côté de la direction géométrique rejoignant lesdits éléments d'extrémité par rapport à une zone des moyens d'ancrage destinée à être fixée auxdites parties osseuses, de sorte que les connexions articulées (5, 6) desdits éléments d'extrémité (2, 3) sont situées entre ladite zone desdits moyens d'ancrage (10,11) et les connexions articulées (8, 9) dudit élément de liaison (7, 14).

7. Implant selon d'une des revendications 5 et 6, **caractérisés en ce que** lesdits éléments de liaison sont des liens rigides (7) tels que des tiges ou barreaux de longueur invariable.

8. Implant selon l'une des revendications 5 et 6, **caractérisé en ce que** ces éléments de liaison (14) comportent eux-mêmes, entre leurs extrémités, au moins une zone déformable (17), ce qui permet alors d'assurer des déplacements, tels qu'un allongement de l'implant sans rotation relative des moyens d'ancrage, par modification de longueur simultanée de l'ensemble comprenant lesdits éléments d'extrémité et l'élément déformable qui leur est relié et de l'élément de liaison (14) et/ou d'assurer un amortissement viscoélastique sans mouvement de rotation desdits moyens d'ancrage.

9. Implant sous forme d'un implant complexe utilisant deux implants individuels selon l'une des revendications 5 à 8 disposés côte à côte, avec une interconnexion hydraulique permettant d'assurer l'une au moins des fonctions suivantes :
mouvement de rotation antisymétrique des moyens de fixation d'un implant individuel par rapport au mouvement des moyens d'ancrage de l'autre implant,
mouvements symétriques desdits moyens de fixation,
mouvements indépendants.

10. Implant squelettique selon l'une des revendications 1 à 9 dans lequel le moyen moteur de l'élément déformable (23, 32, 39, 57) contient un fluide hydraulique indéformable **caractérisé en ce qu'**il comporte des moyens de circuit hydraulique reliés audit élément déformable (23, 32, 39, 57) interposé entre lesdites extrémités, pour permettre une modification durable de la distance entre un élément d'extrémité et un élément (31, 37, 47, 60) mobile par rapport audit élément d'extrémité, lesdits moyens de circuits hydrauliques présentant des moyens de commande, actionnables depuis l'extérieur du corps du patient, ledit élément mobile étant agencé pour provoquer une rotation de l'autre des deux extrémités autour d'un axe sensiblement parallèle audit implant.

11. Implant selon la revendication 10, **caractérisé en ce que** les moyens par lesquels le déplacement dudit élément mobile par le mouvement de l'élément déformable, (23, 32, 57) transforme ce mouvement en un mouvement de rotation dudit autre élément d'extrémité et du moyen d'ancrage qu'elle supporte, est un moyen associant une vis ( 27, 38, 59 ) et un écrou de sorte que le mouvement de translation de l'un provoque un mouvement de rotation de l'autre.

12. Implant selon la revendication 11, **caractérisé en ce que** l'élément déformablé (23, 32, 57) est interposé entre les deux éléments d'extrémité (27, 28, 51, 52) dont l'une est susceptible de tourner, de sorte que la déformation de l'élément déformable (23, 32, 57) entraîne à la fois une rotation et une translation de l'un des éléments d'extrémité, et donc des moyens d'ancrage qu'il porte par rapport à l'autre élément d'extrémité, se traduisant par un mouvement hélicoïdal de l'une des éléments d'extrémité par rapport à l'autre.

13. Implant selon la revendication 10 ou 11, **caractérisé en ce que** les deux éléments d'extrémité (28, 34 ) sont solidaires l'une de l'autre de façon à rester écartées d'une distance invariable, l'élément déformable (39) étant alors interposé entre cet ensemble d'éléments d'extrémité et le moyen mobile (37).

14. Implant squelettique, selon l'une des revendications 1 à 13 **caractérisé en ce que** chacune des deux éléments d'extrémités (27, 28, 51, 52) possède une forme fuselée de façon à ne pas gêner les muscles et les tendons voisins,

15. Implant selon la revendication 14, **caractérisé en ce que** cette forme fuselée comprend une enveloppe (68), appliquée autour de l'implant, dont les deux extrémités, qui présentent des moyens de fixation pour les moyens d'ancrage, émergent de l'enveloppe (68), laquelle contient les différents autres constituants de l'implant, le volume interne libre (67) dans cette enveloppe servant de réservoir de liquide basse pression relié audit élément déformable par une vanne basse pression (66).

16. Implant selon l'une des revendications 1 à 15, **caractérisé en ce qu'**il comporte également un réservoir haute pression (61), de préférence un soufflet, et, également de préférence, un élément déformable différentiel très haute pression (69) pour envoyer du liquide sous haute pression dans le réservoir haute pression (61), le réservoir haute pression (61) étant relié à l'élément déformable (57) également par une vanne haute pression, ledit élément déformable (57) étant relié à un réservoir basse pression (67) par une vanne basse pression (66), les deux vannes haute pression (64) et basse pression (66) étant, de préférence, relativement éloignées l'une de l'autre afin de permettre facilement une commande par des moyens de commande externes, tels que, par exemple, des aimants.

17. Implant complexe, sous forme d'une succession d'implants selon la revendication 14, **caractérisé en ce que**, pour une succession d'implants individuels à divers étages du rachis, on prévoit un réservoir haute pression unique et/ou un moyen de recharge unique des réservoirs hautes pressions, situé à l'écart des différents implants individuels et relié à ceux-ci par des conduits inextensibles.

18. Implant selon l'une des revendications 1 à 17 dans lequel ledit élément déformable (57) contient un fluide hydraulique indéformable et est susceptible d'être alimenté par un réservoir haute pression (61), **caractérisé en ce que** le réservoir de haute pression (61) est conçu pour maintenir le liquide, qu'il contient, sous haute pression, même lorsque des quantités notables de ce liquide sont envoyées aux moyens moteurs de l'implant, provoquant une diminution sensible du volume de liquide, pour maintenir la valeur élevée de pression dans le réservoir haute pression (uuucelui-ci contenant un accumulateur d'énergie (62) sous forme d'une enceinte à paroi élastiquement déformable qui est comprimée, donc diminuée de volume, lorsque le liquide sous haute pression est introduit dans le réservoir haute pression, et qui se détend tout en maintenant une pression élevée lorsque du liquide est soutirée hors du volume haute pression.

19. Implant selon la revendication 18, **caractérisé par** une capsule déformable (62), qui présente un vide important de façon à supprimer tout risque de fuite de gaz.

20. Implant selon l'une des revendications 1 à 19, dans lequel ledit élément déformable est un élément déformable hydraulique (57), **caractérisé en ce qu'**il comporte également un élément déformable hydraulique différentiel très haute pression (69) permettent d'établir une haute pression dans un réservoir haute pression (61) susceptible d'alimenter ledit élément déformable (57) relié auxdites pièces d'extrémités.

21. Implant squelettique selon la revendication 1 **caractérisé en ce que** l'élément déformable comprend au moins un élément de volume déformable (4, 17, 23, 32, 39, 57) sous l'action d'un fluide pour exercer directement ou non une force et/ou un déplacement et/ou un amortissement entre lesdits moyens d'ancrage pour autoriser un mouvement, notamment une rotation, entre lesdits moyens d'ancrage, ledit élément de volume déformable étant capable de recevoir un fluide sous haute pression, un élément de recharge (69) relié à une source de fluide basse pression, ledit élément de recharge (69) étant susceptible d'être actionné par des mouvements de parties corporelles à l'emplacement où il est implanté, pour soustraire du fluide depuis la source de fluide basse pression et augmenter la pression du fluide soustrait de façon à recharger ledit élément de volume déformable en fluide à haute pression.

## Claims

1. Skeletal implant having a first and a second end element (2, 3, 15, 16, 21, 22, 27, 28, 24, 38, 51, 52) of anchoring means (10, 11) capable of being fixed in bone parts, connected respectively to the said first and second end elements, at least one deformable element (4, 17, 23, 32, 39, 57) connected respectively to the said first and second end elements and allowing movement between them and therefore between the said anchoring means, **characterised in that** it comprises articulation means (5, 6, 8, 9, 24, 25, 31, 37, 60) allowing a rotation of the said anchoring means around a perpendicular axis and/or an axis parallel to the geometric direction joining the said end elements, and **in that** it comprises a means for driving the said deformable element in order to cause deformation thereof, resulting in the said rotation.

2. Implant as claimed in Claim 1, **characterised in that** the said driving means is a hydraulic means.

3. Implant as claimed in Claim 1 and 2, **characterised in that** the said deformable element is deformable in rotation.

4. Implant as claimed in Claim 1, **characterised in that** the said deformable member is a bellows supplied from a hydraulic reservoir.

5. Skeletal implant as claimed in one of Claims 1, 2 and 4, **characterised in that** the said anchoring means (10, 11) are connected to the said two ends (2, 3) by articulated connection means (5, 6) and **in that** the said anchoring means (10, 11) are also connected to one another by a connecting element (7, 14) situated at a certain distance from the said end elements, and bearing additional connection means (8, 9), likewise articulated, permitting an angular rotational movement of the said anchoring means (10, 11) relative to one another.

6. Implant as claimed in Claim 5, **characterised in that** the said connecting element (7, 14) on the other side of the geometric direction joining the said end elements with respect to a zone of the anchoring means intended to be fixed to the said bone parts, in such a way that the articulated connections (5, 6) of the said end elements (2, 3) are situated between the said zone of the said anchoring means (10, 11) and the articulated connections (8, 9) of the said connecting element (7, 14).

7. Implant as claimed in one of Claims 5 and 6, **characterised in that** the said connecting elements are rigid links (7) such as rods or bars of variable length.

8. Implant as claimed in one of Claims 5 and 6, **characterised in that** these connecting elements themselves (14) have between their ends at least one deformable zone (17) which then makes it possible to ensure displacements, such as a lengthening of the implant without rotation relative to the anchoring means, by simultaneous modification of the length of the assembly comprising the said end elements and the deformable element which is connected thereto and of the connecting element (14) and/or to ensure viscoelastic damping without rotational movement of the said anchoring means.

9. Implant in the form of a complex implant using two individual implants as claimed in one of Claims 5 to 8 disposed side by side, with a hydraulic interconnection making it possible to ensure at least one of the following functions:
- asymmetrical rotational movement of the fixing means of an individual implant relative to the movement of the anchoring means of the other implant,
- symmetrical movements of the said fixing means,
- independent movement.

10. Skeletal implant as claimed in one of Claims 1 to 9, in which the driving means of the deformable element (23, 32, 39, 57) contains a non-deformable hydraulic fluid, **characterised in that** it includes hydraulic circuit means connected to the said deformable element (23, 32, 39, 57) interposed between the said ends, in order to permit a lasting modification of the distance between an end element and an element (31, 37, 47, 60) which is movable relative to the said end element, the said hydraulic circuit means having control means which can be actuated from outside the patient's body, the said movable element being arranged so as to cause a rotation of the other of the two ends about an axis substantially parallel to the said implant.

11. Implant as claimed in Claim 10, **characterised in that** the means by which the displacement of the said movable element by the movement of the deformable element (23, 32, 57) transforms this movement into a rotational movement of the said other end element and of the anchoring means which it supports, is a means associating a screw (27, 38, 59) and a nut in such a way that the translational movement of one causes a rotational movement of the other.

12. Implant as claimed in Claim 11, **characterised in that** the deformable element (23, 32, 57) is interposed between the two end elements (27, 28, 51, 52), one of which is capable of turning, in such a way that the deformation of the deformable element (23, 32, 57) results simultaneously in a rotation and a translation of one of the end elements, and therefore of the anchoring means which it bears, relative to the other end element, resulting in a helical movement of one of the end elements relative to the other.

13. Implant as claimed in Claims 10 or 11, **characterised in that** the two end elements (28, 34) are joined to one another in such a way as to remain spaced by an invariable distance, the deformable element (39) then being interposed between this assembly of end elements and the movable means (37).

14. Skeletal implant as claimed in one of Claims 1 to 13, **characterised in that** each of the two end elements (27, 28, 51, 52) has a streamlined shape so as not to disturb the neighbouring muscles and tendons.

15. Implant as claimed in Claim 14, **characterised in that** this streamlined shape comprises a sheath (68) which is applied around the implant and of which the two ends having fixing means for the anchoring means emerge from the sheath (68) which contains the various other components of the implant, the free internal volume (67) in this sheath serving as a reservoir of low-pressure liquid connected to the said deformable element by a low-pressure valve.

16. Implant as claimed in one of Claims 1 to 15, **characterised in that** it also comprises a high-pressure reservoir (61), preferably a bellows, and, equally preferably, a very high-pressure differential deformable element (69) for sending liquid under high pressure into the high-pressure reservoir (61), the high-pressure reservoir (61) being connected to the deformable element (57) likewise by a high-pressure valve, the said deformable element (57) being connected to a low-pressure reservoir (67) by a low-pressure valve (66), the two high-pressure and low-pressure valves (64, 66) being preferably relatively far apart from one another in order easily to permit control by external control means, such as for example magnets.

17. Complex implant in the form of a succession of implants as claimed in Claim 14, **characterised in that**, for a succession of individual implants at various stages of the rachis, there is provided a single high-pressure reservoir and/or a single means for recharging the high-pressure reservoirs, situated spaced from the various individual implants and connected to them by inextensible conduits.

18. Implant as claimed in one of Claims 1 to 17, in which the said deformable element (57) contains a non-deformable hydraulic fluid and is capable of being supplied by a high-pressure reservoir (61), **characterised in that** the high-pressure reservoir (61) is designed to keep the liquid which it contains under high pressure, even when considerable quantities of this liquid are sent to the driving means of the implant, causing a substantial decrease in the volume of liquid in order to maintain the raised pressure value in the high-pressure reservoir, this latter containing an energy store (62) in the form of a chamber with resiliently deformable wall which is compressed, and therefore reduced in volume, when the liquid under high pressure is introduced into the high-pressure reservoir, and which expands whilst maintaining a raised pressure when liquid is withdrawn from the high-pressure volume.

19. Implant as claimed in Claim 18, **characterised by** a deformable capsule (62) which has a substantial vacuum so as to eliminate any risk of escape of gas.

20. Implant as claimed in one of Claims 1 to 19, in which the said deformable element is a hydraulic deformable element (57), **characterised in that** it also comprises a very high-pressure differential hydraulic deformable element (69) which makes it possible to establish a high pressure in a high-pressure reservoir (61) capable of supplying the said deformable element (57) connected to the said end parts.

21. Skeletal implant as claimed in Claim 1, **characterised in that** the deformable element comprises at least one element (4, 17, 23, 32, 39, 57) with a volume deformable under the action of a fluid so as to exert directly or indirectly a force and/or a displacement and/or a damping between the said anchoring means in order to allow a movement, particularly rotation, between the said anchoring means, the said element of deformable volume being capable of receiving a fluid under high pressure, a recharging element (69) connected to a source of low-pressure fluid, the said recharging element (69) being capable of being actuated by movements of body parts at the site where it is implanted, in order to extract fluid from the source of low-pressure fluid and to increase the pressure of the fluid extracted in such a way as to recharge the said element of deformable volume with high-pressure fluid.

## Patentansprüche

1. Skelettimplantat, das ein erstes und ein zweites Endelement (2, 3, 15, 16, 21, 22, 27, 28, 34, 38, 51, 52), Verankerungsmittel (10, 11), die in Knochenteilen befestigt werden können und die mit dem ersten bzw. dem zweiten Endelement verbunden sind, und mindestens ein verformbares Element (4, 17, 23, 32, 39, 57) aufweist, das mit dem ersten bzw. mit dem zweiten Endelement verbunden ist und zwischen ihnen und damit zwischen den Verankerungsmitteln eine Bewegung zuläßt, **dadurch gekennzeichnet, daß** es Gelenkmittel (5, 6, 8, 9, 24, 25, 31, 37, 60) aufweist, die eine Drehung der Verankerungsmittel um eine Achse zulassen, die zu der geometrischen Richtung, die mit den Endelementen zusammenfällt, senkrecht und/oder parallel ist, und daß es Mittel zum Antreiben des verformbaren Elements aufweist, um seine Verformung zu bewirken, die diese Drehung mit sich bringt.

2. Implantat nach Anspruch 1, **dadurch gekennzeichnet, daß** das Antriebsmittel ein hydraulisches Mittel ist.

3. Implantat nach Anspruch 1 und 2, **dadurch gekennzeichnet, daß** das verformbare Element in Drehung verformbar ist.

4. Implantat nach Anspruch 1, **dadurch gekennzeichnet, daß** das verformbare Organ ein Balg ist, der aus einem Hydraulikbehälter versorgt wird.

5. Skelettimplantat nach einem der Ansprüche 1, 2 und 4, **dadurch gekennzeichnet, daß** die Verankerungsmittel (10, 11) mit den beiden Enden (2, 3) durch gelenkige Verbindungsmittel (5, 6) verbunden sind und daß die Verankerungsmittel (10, 11) außerdem miteinander durch ein Verbindungselement (7, 14) verbunden sind, das in einem gewissen Abstand von den Endelementen angeordnet ist und zusätzliche, ebenfalls gelenkige Verbindungsmittel (8, 9) trägt, die eine Drehwinkelbewegung der Verankerungsmittel (10, 11) zueinander gestatten.

6. Implantat nach Anspruch 5, **dadurch gekennzeichnet, daß** das Verbindungselement (7, 14) bezüglich einer Zone der Verankerungsmittel, die dazu bestimmt ist, an den Knochenteilen befestigt zu werden, auf der anderen Seite der mit den Endelementen zusammenfallenden geometrischen Richtung angeordnet ist, so daß die gelenkigen Verbindungen (5, 6) der Endelemente (2, 3) zwischen dieser Zone der Verankerungsmittel (10, 11) und den gelenkigen Verbindungen (8, 9) des Verbindungselements (7, 14) angeordnet sind.

7. Implantat nach einem der Ansprüche 5 und 6, **dadurch gekennzeichnet, daß** die Verbindungselemente starre Bindeglieder (7) wie Stangen oder Stäbe von unveränderlicher Länge sind.

8. Implantat nach einem der Ansprüche 5 und 6, **dadurch gekennzeichnet, daß** diese Verbindungselemente (14) ihrerseits zwischen ihren Enden mindestens eine verformbare Zone (17) aufweisen, was nun die Durchführung von Bewegungen, wie eine Verlängerung des Implantats ohne Relativdrehung der Verankerungsmittel durch gleichzeitige Längenänderung der Einheit, die die Endelemente und das mit ihnen verbundene verformbare Element umfaßt, und des Verbindungselements (14) gestattet und/oder eine viskoelastische Dämpfung ohne Drehbewegung der Verankerungsmittel gestattet.

9. Implantat in Form eines komplexen Implantats, das zwei nebeneinander angeordnete Einzelimplantate nach einem der Ansprüche 5 bis 8 mit einer gegenseitigen hydraulischen Verbindung benutzt und mindestens eine der folgenden Funktionen gewährleistet:
antisymmetrische Drehbewegung der Befestigungsmittel eines Einzelimplantats bezüglich der Bewegung der Verankerungsmittel des anderen Implantats,
symmetrische Bewegungen der Befestigungsmittel,
unabhängige Bewegungen.

10. Skelettimplantat nach einem der Ansprüche 1 bis 9, bei dem das Mittel zum Antrieb des verformbaren Elements (23, 32, 39, 57) ein unverformbares Hydraulikfluid enthält, **dadurch gekennzeichnet, daß** es Hydraulikkreismittel aufweist, die mit dem zwischen diese Enden eingesetzten verformbaren Element (23, 32, 39, 57) verbunden sind, um eine dauerhafte Änderung des Abstands zwischen einem Endelement und einem bezüglich diesem Endelement beweglichen Element (31, 37, 47, 60) zu gestatten, wobei die Hydraulikkreismittel Steuermittel aufweisen, die von außerhalb des Körpers des Patienten betätigbar sind, wobei das bewegliche Element ausgebildet ist, um eine Drehung des anderen der beiden Enden um eine zum Implantat im wesentlichen parallele Achse zu bewirken.

11. Implantat nach Anspruch 10, **dadurch gekennzeichnet, daß** die Mittel, durch die die Bewegung des beweglichen Elements durch die Bewegung des verformbaren Elements (23, 32, 57) diese Bewegung in eine Drehbewegung des anderen Endelelements und des Verankerungsmittels, das es trägt, umwandelt, ein eine Schraube (27, 38, 59) und eine Mutter kombinierendes Mittel ist, so daß die Translationsbewegung des einen eine Drehbewegung des anderen bewirkt

12. Implantat nach Anspruch 11, **dadurch gekennzeichnet, daß** das verformbare Element (23, 32, 57) zwischen die beiden Endelemente (27, 28, 51, 52) eingesetzt ist, von denen eines sich drehen kann, so daß die Verformung des verformbaren Elements (23, 32, 57) gleichzeitig eine Drehung und eine Translation des einen der Endelemente und damit der Verankerungsmittel, die es trägt, bezüglich des anderen Endelements mit sich bringt, was sich in einer schraubenförmigen Bewegung des einen der Endelemente bezüglich des anderen äußert.

13. Implantat nach Anspruch 10 oder 11, **dadurch gekennzeichnet, daß** die beiden Endelemente (28, 34) miteinander fest verbunden sind, so daß sie in einem unveränderlichen Abstand voneinander entfernt bleiben, wobei das verformbare Element (39) nun zwischen diese Einheit von Endelementen und das bewegliche Mittel (37) eingesetzt sind.

14. Skelettimplantat nach einem der Ansprüche 1 bis 13, **dadurch gekennzeichnet, daß** jedes der beiden Endelemente (27, 28, 51, 52) eine Spindelform besitzt, so daß es die benachbarten Muskeln und Sehnen nicht stört.

15. Implantat nach Anspruch 14, **dadurch gekennzeichnet, daß** diese Spindelform eine Hülle (68) aufweist, die um das Implantat herum aufgelegt ist, deren beide Enden, die Befestigungsmittel für die Verankerungsmittel aufweisen, aus der Hülle (68) hervorstehen, die die verschiedenen anderen Bestandteile des Implantats enthält, wobei das freie Innenvolumen (67) in dieser Hülle als Behälter für Niederdruckflüssigkeit dient, das mit dem verformbaren Element über ein Niederdruckventil (66) verbunden ist.

16. Implantat nach einem der Ansprüche 1 bis 15, **dadurch gekennzeichnet, daß** es ferner einen HochdruckBehälter (61), vorzugsweise einen Balg, und ebenfalls vorzugsweise ein verformbares Differenzelement von sehr hohem Druck (69) aufweist, um Flüssigkeit unter hohem Druck in den HochdruckBehälter einzuspeisen, wobei der HochdruckBehälter (61) mit dem verformbaren Element (57) ebenfalls über ein Hochdruckventil verbunden ist, wobei das verformbare Element (57) mit einem NiederdruckBehälter (67) über ein Niederdruckventil (66) verbunden ist, wobei das Hochdruckventil (64) und das Niederdruckventil (66) vorzugsweise voneinander relativ entfernt sind, um leicht eine Steuerung durch externe Mittel, wie z.B. Magnete, zu gestatten.

17. Komplexes Implantat in Form einer Folge von Implantaten nach Anspruch 14, **dadurch gekennzeichnet, daß** man für eine Folge von Einzelimplantaten in verschiedenen Stufen der Wirbelsäule einen einzigen HochdruckBehälter und/oder ein einziges Mittel zum Nachfüllen der HochdruckBehälter vorsieht, das in einem Abstand von den einzelnen Einzelimplantaten angeordnet ist und mit diesen durch unausdehnbare Leitungen verbunden ist.

18. Implantat nach einem der Ansprüche 1 bis 17, bei dem das verformbare Element (57) ein unverformbares Hydraulikfluid enthält und durch einen HochdruckBehälter (61) versorgt werden kann, **dadurch gekennzeichnet, daß** der HochdruckBehälter (61) dafür ausgelegt ist, die Flüssigkeit, die er enthält, unter hohem Druck zu halten, selbst wenn beträchtliche Mengen dieser Flüssigkeit den Antriebsmitteln des Implantats zugeführt werden, was eine beträchtliche Verringerung des Flüssigkeitsvolumens verursacht, um den hohen Druckwert in dem Hochdruckbehälter aufrecht zu erhalten (wobei dieser einen Energiespeicher (62) in Form einer Kammer mit elastisch verformbarer Wand aufweist, die komprimiert und damit im Volumen verkleinert wird, wenn die Flüssigkeit unter hohem Druck in den Hochdruckbehälter eingeführt wird, und die sich entspannt, indem gleichzeitig ein hoher Druck aufrecht erhalten wird, wenn Flüssigkeit aus dem Hochdruckvolumen abgezogen wird.

19. Implantat nach Anspruch 18, **gekennzeichnet durch** eine verformbare Kapsel (62), die ein starkes Vakuum aufweist, so daß jede Gefahr des Austritts von Gas unterdrückt wird.

20. Implantat nach einem der Ansprüche 1 bis 19, bei dem das verformbare Element ein hydraulisches verformbares Element (57) ist, **dadurch gekennzeichnet, daß** es ferner ein hydraulisches Differenzelement(69) von sehr hohem Druck aufweist, das gestattet, einen hohen Druck in einem Hochdruckbehälter (61) herzustellen, der das verformbare Element (57), das mit den Endteilen verbunden ist, versorgen kann.

21. Skelettimplantat nach Anspruch 1, **dadurch gekennzeichnet, daß** das verformbare Element mindestens ein Element (4, 17, 23, 32, 39, 57) mit unter der Einwirkung eines Fluids verformbarem Volumen aufweist, um direkt oder nicht eine Kraft und/oder eine Bewegung und/oder eine Dämpfung zwischen den Verankerungsmitteln auszuüben, um eine Bewegung, insbesondere eine Drehung, zwischen den Verankerungsmitteln zuzulassen, wobei das Element mit verformbarem Volumen ein Fluid unter hohem Druck aufnehmen kann, wobei ein Nachfüllelement (69) mit einer Niederdruckfluidquelle verbunden ist, wobei dieses Nachfüllelement (69) durch Bewegungen von Körperteilen an der Stelle, an der es implantiert ist, betätigt werden kann, um von der Niederdruckfluidquelle Fluid abzuziehen und den Druck des abgezogenen Fluids zu erhöhen, so daß das Element mit verformbarem Volumen mit Hochdruckfluid nachgefüllt wird.
